# EUROPEAN PATENT SPECIFICATION

(11) **EP 2 904 118 B1**
(45) Date of publication and mention of the grant of the patent: **20.03.2019**
(21) Application number: 13844293.4
(22) Date of filing: 02.10.2013
(51) Int. Cl.: C12Q 1/6883

(54) **URINE EXOSOME MRNAS AND METHODS OF USING SAME TO DETECT DIABETIC NEPHROPATHY**
EXOSOME URIN-MRNAS UND VERFAHREN ZUR VERWENDUNG DAVON ZUR ERKENNUNG VON DIABETISCHER NEPHROPATHIE
ARNM D'EXOSOME D'URINE ET PROCÉDÉS D'UTILISATION DE CEUX-CI POUR DÉTECTER UNE NÉPHROPATHIE DIABÉTIQUE

(30) Priority: 05.10.2012 US 201261710627 P
(43) Date of publication of application: 12.08.2015
(73) Proprietor: Hitachi Chemical Co., Ltd., Chiyoda-ku Tokyo 100-6606 (JP); Hitachi Chemical Co. America, Ltd., Cupertino, CA 95014 (US); THE REGENTS OF THE UNIVERSITY OF CALIFORNIA, Oakland, CA 94607-5200 (US)
(72) Inventor: MITSUHASHI, Masato, Irvine, CA 92614 (US); SHARMA, Kumar, Del Mar, CA 92014 (US)
(74) Representative: Vossius & Partner Patentanwälte Rechtsanwälte mbB
(86) International application number: PCT/US2013/063122
(87) International publication number: WO 2014/055687

(56) References cited:
- EP-A1- 1 672 062
- WO-A1-2011/156763
- WO-A1-2011/156763
- GUNTER MÜLLER: "Microvesicles/exosomes as potential novel biomarkers of metabolic diseases", DIABETES, METABOLIC SYNDROME AND OBESITY: TARGETS AND THERAPY, 1 August 2012 (2012-08-01), page 247, XP055089998, DOI: 10.2147/DMSO.S32923
- KEVIN C MIRANDA ET AL: "Nucleic acids within urinary exosomes/microvesicles are potential biomarkers for renal disease", KIDNEY INTERNATIONAL, vol. 78, no. 2, 28 April 2010 (2010-04-28) , pages 191-199, XP055107901, ISSN: 0085-2538, DOI: 10.1038/ki.2010.106
- CRISTINA BELTRAMI ET AL: "Analysis of urinary microRNAs in chronic kidney disease: Figure 1", BIOCHEMICAL SOCIETY TRANSACTIONS, vol. 12, no. 4, 1 August 2012 (2012-08-01) , pages 4-879, XP055266737, GB ISSN: 0300-5127, DOI: 10.1093/nar/gkq601
- S. MATHIVANAN ET AL: "ExoCarta 2012: database of exosomal proteins, RNA and lipids", NUCLEIC ACIDS RESEARCH, vol. 40, no. D1, 11 October 2011 (2011-10-11), pages D1241-D1244, XP055266784, GB ISSN: 0305-1048, DOI: 10.1093/nar/gkr828
- BARNETT, A ET AL.: 'Angiotensin-Receptor Blockade Versus Converting-Enzyme Inhibition In Type 2 Diabetes and Nephropathy.' THE NEW ENGLAND JOURNAL OF MEDICINE. vol. 351, 04 November 2004, pages 1952 - 1961, XP009079340
- ZHENG, M ET AL.: 'Urinary Podocyte-Associated mRNA profile in Various Stages of Diabetic Nephropathy.' PLOS ONE. vol. 6, 31 May 2011, pages 1 - 7, XP055246548

## Description

### BACKGROUND

### Field

The present disclosure relates to generally identification of various biomarkers associated with diabetic nephropathy. Several embodiments relate to the diagnosis of diabetic nephropathy and more specifically, the present disclosure relates to the identification and use of mRNAs isolated from exosomes derived from urine and their use in the recognition and ongoing monitoring of diabetic nephropathy.

### Description of Related Art

Broadly speaking, the kidney functions to filter the blood of various metabolic waste products and excess water. Every day, an adult's kidneys filter about 200 quarts of blood resulting in generation and excretion of about 2 quarts of waste products and extra water. The functional unit of the kidney is the nephron, and each kidney comprises about 1 million nephrons. Within each nephron is a glomerulus, which acts as the filtering mechanism, which keeps normal proteins and/or cells in the blood stream, and allows the excess water and waste to pass through to be processed by the remainder of the nephron (e.g., either concentrated or diluted). While some loss of kidney function can be tolerated - many individuals can lead normal lives with just one kidney - in many cases the cause of the reduction in kidney function is due to progressive disease or damage. In extreme cases, the progressive loss of kidney function results in the need for renal replacement therapy, such as dialysis or even kidney transplant. Two of the most common causes of loss of kidney function are diabetes and high blood pressure. According to recent data from the American Diabetes Association, over 8% of the United States population is diabetic. Over 200,000 people were living on chronic dialysis or with kidney transplant, due to end-stage kidney disease caused by diabetes. This comes at an extraordinary cost, not only to the patient's themselves, but to their family and the healthcare system.

Previously, a method of detecting proliferative diseases causing sclerosis, comprising measuring the expression of at least one substance selected from the group consisting of STAT3, phosphorylated STAT3, Smad1, phosphorylated Smad1, activin receptor-like kinase 1, activin receptor-like kinase 3 and bone morphogenetic proteins in a biological sample has been described; EP 1 672 062 A1.

Moreover, methods of characterizing kidney function have previously been described focussing the quantification of kidney-associated marker RNA isolated from vesicles contained in patient urine samples; WO 2011/156763 A1.

G. Müller previously described microvesicles/exosomes as potential novel biomarkers of metabolic diseases; G. Müller, Diabetes, Metabolic Syndrome and Obesity 5:247-282 (2012). Further, nucleic acids within urinary exosomes/microvesicles have previously been described as potential biomarkers for renal diseases; Miranda et al., Kidney International 78(2):191-199 (2010).

Moreover, the analysis of urinary microRNAs in chronic kidney disease has previously been described; Beltrami et al., Biochemical Society Transactions 12(4):875-879 (2012).

In addition, the "ExoCarta 2012: database of exosomal proteins, RNA and lipids" has previously been published; Mathivanan et al., Nucleic Acid Research 40(D1): D1241-D1244 (2011).

### SUMMARY

The present invention is defined in the claims. Thus, it relates to the following items:
1. A method for identifying a subject currently affected by diabetic nephropathy due to Type I or Type II diabetes, comprising:
   isolating vesicles that are associated with RNA from a sample of urine that has been obtained from a subject, wherein said sample comprises vesicles that are associated with RNA;
   lysing said vesicles to release said vesicle-associated RNA, wherein said vesicle-associated RNA comprises an RNA associated with diabetes-induced damage to the nephron,
   wherein said RNA associated with diabetes-induced damage to the nephron is selected from the group consisting of PPARGC1A, SMAD1, NRF2, UMOD, SLC12A1 and CD24;
   quantifying said RNA associated with diabetes-induced damage to the nephron by a method comprising:
      (i) contacting said RNA from said sample with a reverse transcriptase to generate complementary DNA (cDNA), and
      (ii) contacting said cDNA with sense and antisense primers that are specific for one of PPARGC1A, SMAD1, NRF2, UMOD, SLC12A1 and CD24 and a DNA polymerase to generate amplified DNA;
   comparing the amount of quantified RNA associated with diabetes-induced damage to the nephron from said sample to a quantity of a corresponding RNA from individuals having normal kidney function; and
   identifying a subject currently affected by diabetic nephropathy due to Type I or Type II diabetes when there is a difference between the quantity of said RNA associated with diabetes-induced damage to the nephron in said subject and said quantity of the RNA in individuals with normal kidney function.
2. The method of item 1, wherein the difference in the quantity of RNA associated with diabetes-induced damage to the nephron is correlated with one or more non-molecular indicators of diabetic nephropathy.
3. The method according to item 1 or 2, wherein said RNA associated with diabetes-induced damage to the nephron is selected from the group consisting of PPARGCIA, SMAD1, UMOD and SLC12A1.
4. The method according to any one of items 1 to 3, wherein isolating the vesicles from said sample comprises filtering the urine, wherein said filtration traps said vesicles on the filter.
5. The method of item 4, wherein said lysing is performed while said vesicles are trapped on said filter.
6. The method according to any one of items 1 to 5, further comprising centrifuging said sample to remove cellular debris, wherein said centrifugation is performed prior to isolating the vesicles.
7. The method of item 6, wherein concentrating the vesicles further comprises filtering the supernatant of said centrifuged urine.
8. The method according to any one of items 1 to 7, wherein said Type I or Type II diabetes has not yet been diagnosed.
9. The method according to any one of items 1 to 8, wherein said RNA associated with diabetes-induced damage to the nephron comprises poly(A)+ RNA.
10. A method according to any one of the preceding items, wherein the method is used to screen a plurality of subjects to determine their likelihood of developing diabetic nephropathy due to Type I or Type II diabetes and/or to detect early stage diabetic nephropathy due to Type I or Type II diabetes.
11. A method for identifying a subject affected by diabetic nephropathy due to Type I or Type II diabetes, comprising:
   isolating vesicles that are associated with RNA from a sample of urine that has been obtained from a subject, wherein said sample comprises vesicles that are associated with RNA;
   lysing said vesicles to release said vesicle-associated RNA, wherein said vesicle-associated RNA comprises an RNA associated with diabetes-induced damage to the nephron,
   wherein said RNA associated with diabetes-induced damage to the nephron is selected from the group consisting of PPARGC1A, SMAD1, NRF2, UMOD, SLC12A1 and CD24;
   quantifying said RNA associated with diabetes-induced damage to the nephron such as by using a method selected from the group consisting of RNA sequencing, RNA microarray, nucleic acid sequence-based amplification, northern blotting, RNase protection assay and branched-DNA amplification; and
   comparing the amount of said RNA associated with diabetes-induced damage to the nephron from said subject to the quantity of a corresponding RNA from individuals having normal kidney function, wherein a difference in the quantity of said RNA associated with diabetes-induced damage to the nephron between said subject and said individuals indicates the subject is affected by diabetic nephropathy due to Type I or Type II diabetes.
12. Use of a kit for detection of early stage diabetic nephropathy, comprising:
   (a) a reverse transcriptase enzyme for generating complementary DNA (cDNA) from RNA isolated from a urine sample of a subject being evaluated for their diabetic nephropathy status;
   (b) at least one pair of sense and antisense primers specific for one of PPARGC1A, UMOD, NRF2, SLC12A1 and CD24; and
   (c) a DNA polymerase for generating amplified DNA from the cDNA.
13. The use of the kit of item 12, wherein said kit further comprises a filter for capturing vesicles from said urine sample.
14. The use of the kit of item 13, wherein said kit further comprises a lysis buffer for liberating RNA from said vesicles.
15. The use of the kit of item 14, wherein said kit further comprises an elution buffer for transporting RNA from said lysed vesicles to an analysis vessel.
16. The use of the kit according to any one of items 12 to 15, wherein said kit further comprises a microplate configured to receive said RNA.
17. The use of the kit of item 16, wherein said microplate comprises oligo(dT) in each well of the plate.
18. The use of the kit according to any one of items 12 to 17, wherein said kit further comprises a DNA amplification buffer comprising Tris-HCl, magnesium chlorides, potassium chloride, and adenine, thymine, guanine, and cytosine nucleotides.
19. The use of the kit according to any one of items 12 to 18, wherein said kit further comprises at least one fluorescent probe complementary to a region within the amplified DNA of one of PPARGC1A, SMAD1, UMOD, NRF2, SLC12A1 and CD24.
20. The use of the kit of item 19, wherein said kit further comprises a means for detecting said fluorescent probe.
21. The use of the kit of any one of items 12 to 20, wherein the use is for the detection of diabetes-induced damage to the nephron.
22. The use of the kit of item 21, wherein the diabetes-induced damage to the nephron is correlated with one or more non-molecular indicators of diabetic nephropathy.
23. The use of the kit of any one of items 12 to 22, wherein the kit further comprises control DNA indicating expression levels of one of PPARGC1A, SMAD1, UMOD, NRF2, SLC12A1 and CD24 in the absence of diabetic nephropathy.
24. The use of the kit of any one of items 12 to 23, wherein the use is for detecting diabetic nephropathy that is due to Type I or Type II diabetes.

Thus, in the context of the present invention, there is disclosed a method for identifying a subject likely to develop or currently affected by diabetic nephropathy, the method comprising obtaining a sample of urine from a subject, wherein the sample comprises vesicles that are associated with RNA, isolating the vesicles from the sample, lysing the vesicles to release the vesicle-associated RNA, wherein the vesicle-associated RNA comprises an RNA associated with diabetes-induced damage to the nephron, wherein the RNA associated with diabetes-induced damage to the nephron is selected from the group consisting of PPARGC1A, SMAD1, UMOD, NRF2, SLC12A1 and CD24, quantifying the RNA associated with diabetes-induced damage to the nephron, comparing the amount of the RNA associated with diabetes-induced damage to the nephron from the subject to the quantity of a corresponding RNA from individuals having normal kidney function, and identifying a subject as likely to develop or currently affected by diabetic nephropathy when there is a difference in the quantity of the RNA associated with diabetes-induced damage to the nephron between the subject and the quantity of the RNA in individuals with normal kidney function. The quantifying may be performed by a method comprising contacting RNA from the sample with a reverse transcriptase to generate complementary DNA (cDNA) and (ii) contacting the cDNA with sense and antisense primers that are specific for one of PPARGC1A, SMAD1, UMOD, NRF2, SLC12A1 and CD24 and a DNA polymerase to generate amplified DNA. Thereafter, the expression levels of the mRNA can be quantified (using the amplified DNA as a surrogate).

The methods may further comprise administering a therapy to subject, based on the outcome of the results. For example, a drug therapy may be administered, either alone or in conjunction with diet, exercise, or lifestyle changes. In addition, dialysis may also be administered. It is also disclosed that a kidney transplant is performed.

There is also provided a method for identifying a subject affected by diabetic nephropathy, comprising obtaining a sample of urine from a subject, wherein the sample comprises vesicles that are associated with RNA, isolating the vesicles from the sample, lysing the vesicles to release the vesicle-associated RNA, wherein the vesicle-associated RNA comprises an RNA associated with diabetes-induced damage to the nephron, wherein the RNA associated with diabetes-induced damage to the nephron is selected from the group consisting of PPARGC1A, SMAD1, UMOD, NRF2, SLC12A1 and CD24, quantifying the RNA associated with diabetes-induced damage to the nephron , and comparing the amount of the RNA associated with diabetes-induced damage to the nephron from the subject to the quantity of a corresponding RNA from individuals having normal kidney function, wherein a difference in the quantity of the RNA associated with diabetes-induced damage to the nephron between the subject and the individuals indicates the subject is affected by diabetic nephropathy.

Moreover, there is additionally disclosed a method for determining the progression of diabetic nephropathy in a patient comprising, obtaining a first sample of urine from a patient at a first time and a second sample of urine from the patient at a second time that is after the first time; wherein the samples comprise vesicles that are associated with RNA, isolating the vesicles from the samples, lysing the vesicles to release the vesicle-associated RNA, wherein the vesicle-associated RNA comprises at least one RNA associated with diabetes-induced damage to the nephron and at least one RNA that does not change in response to diabetes-induced damage to the nephron, wherein the at least one RNA associated with diabetes-induced damage to the nephron is selected from the group consisting of PPARGC1A, SMAD1, UMOD, NRF2, SLC12A1, CD24, and combinations thereof, quantifying the at least one RNA associated with diabetes-induced damage to the nephron and the at least one RNA that does not change in response to diabetes-induced damage to the nephron, and determining a ratio between the amounts of the at least one RNA associated with diabetes-induced damage to the nephron and the at least one RNA that does not change in response to diabetes-induced damage to the nephron, and identifying progression in the patient's diabetic nephropathy when the ratio of the at least one RNA one RNA associated with diabetes-induced damage to the nephron to the at least one RNA that does not change in response to diabetes-induced damage to the nephron is increased in the second sample as compared to the first sample.

There is additionally disclosed a nucleic-acid based method for detection of early stage diabetic nephropathy, comprising obtaining a sample of urine from a subject, wherein the sample comprises vesicles that are associated with RNA, isolating the vesicles from the sample, lysing the vesicles to release the vesicle-associated RNA, wherein the vesicle-associated RNA comprises an RNA associated with diabetes-induced damage to the nephron, wherein the RNA associated with diabetes-induced damage to the nephron is selected from the group consisting of PPARGC1A, SMAD1, UMOD, NRF2, SLC12A1 and CD24, quantifying the RNA associated with diabetes-induced damage to the nephron, and comparing the amount of the RNA associated with diabetes-induced damage to the nephron from the subject to the quantity of a corresponding RNA from individuals having normal kidney function, wherein a difference in the quantity of the RNA associated with diabetes-induced damage to the nephron between the subject and the individuals indicates early stage diabetic nephropathy, thereby detecting early stage diabetic nephropathy. In several embodiments, the detection can be achieved prior to detection by non-nucleic acid detection methods.

There is also disclosed a method for advising a subject to undertake a therapy regime for diabetic nephropathy, comprising ordering a test of the subject's urine, the test comprising obtaining a sample of urine from a subject, wherein the sample comprises vesicles that are associated with RNA, isolating the vesicles from the sample, lysing the vesicles to release the vesicle-associated RNA, wherein the vesicle-associated RNA comprises an RNA associated with diabetes-induced damage to the nephron, wherein the RNA associated with diabetes-induced damage to the nephron is selected from the group consisting of PPARGC1A, SMAD1, UMOD, NRF2, SLC12A1 and CD24, quantifying the RNA associated with diabetes-induced damage to the nephron, comparing the amount of the RNA associated with diabetes-induced damage to the nephron from the subject to the quantity of a corresponding RNA from individuals having normal kidney function, characterizing the subject as likely to develop or currently affected by diabetic nephropathy when there is a difference in the quantity of the RNA associated with diabetes-induced damage to the nephron between the subject and the quantity of the RNA in individuals with normal kidney function, and advising the subject to undertake a therapy for diabetic nephropathy when characterized as likely to develop or currently affected by diabetic nephropathy. As disclosed herein, the therapy comprises one or more of diet, exercise, dialysis and/or lifestyle changes.

There is additionally disclosed a method for treating a subject having diabetic nephropathy comprising obtaining a sample of urine from a subject, wherein the sample comprises vesicles that are associated with RNA, isolating the vesicles from the sample, lysing the vesicles to release the vesicle-associated RNA, wherein the vesicle-associated RNA comprises an RNA associated with diabetes-induced damage to the nephron, wherein the RNA associated with diabetes-induced damage to the nephron is selected from the group consisting of PPARGC1A, SMAD1, UMOD, NRF2, SLC12A1 and CD24, quantifying the RNA associated with diabetes-induced damage to the nephron, comparing the amount of the RNA associated with diabetes-induced damage to the nephron from the subject to the quantity of a corresponding RNA from individuals having normal kidney function, characterizing the subject as currently affected by diabetic nephropathy when there is a difference in the quantity of the RNA associated with diabetes-induced damage to the nephron between the subject and the quantity of the RNA in individuals with normal kidney function, and administering a therapy to the to treat the diabetic nephropathy. In several embodiments, the therapy comprises one or more of diet, exercise, dialysis and/or lifestyle changes.

In several embodiments, the quantifying of the RNA is achieved by using a method selected from the group consisting of reverse-transcription polymerase chain reaction (RT-PCR), real-time RT-PCR, RNA sequencing, northern blotting, fluorescence activated cell sorting, ELISA, and mass spectrometry. Other quantification methods may also optionally be used. In several embodiments, the quantifying comprises use of real-time RT-PCR.

In several embodiments, differences in the quantity of an RNA associated with diabetes-induced damage to the nephron are correlated with one or more non-molecular indicators of diabetic nephropathy. In such embodiments, an initial, molecular diagnosis can be corroborated through the use of non-molecular means.

In several embodiments, the RNA associated with diabetes-induced damage to the nephron is selected from the group consisting of PPARGC1A, SMAD1, UMOD, and SLC12A1. In several embodiments, these markers are not otherwise expressed or detectable until identified using the methods disclosed herein, thereby allowing early detection of damage to the nephron (e.g., prior to manifestation of established symptoms).

In several embodiments, the isolation of the vesicles from the sample comprises filtering the urine. In several embodiments, the filtration traps the vesicles on the filter. The lysing is performed while the vesicles are trapped on the filter.

In several embodiments, the methods further comprise centrifuging the urine sample (or samples) to remove cellular debris. In several embodiments, centrifugation is performed prior to isolating the vesicles. In several embodiments, the methods comprise concentrating the vesicles further by filtering the supernatant of the centrifuged urine.

The diabetic nephropathy is due to Type I or Type II diabetes. In some embodiments, the Type I or Type II diabetes has not yet been diagnosed.

In several embodiments, the RNA associated with diabetes-induced damage to the nephron comprises poly(A)+ RNA.

In several embodiments, the methods disclosed herein are used to screen a plurality of subjects to determine their likelihood of developing diabetic nephropathy and/or to detect early stage diabetic nephropathy. As disclosed herein, the methods may further comprise treating the subjects for prevent and/or treat diabetic nephropathy.

Additionally, there are provided, in several embodiments, kits for detection of diabetic nephropathy. For example, in several embodiments, there is provided a kit for detection of early stage diabetic nephropathy, comprising (a) a reverse transcriptase enzyme for generating complementary DNA (cDNA) from RNA isolated from a urine sample of a subject being evaluated for their diabetic nephropathy status, (b) at least one pair of sense and antisense primers specific for one of PPARGC1A, SMAD1, UMOD, NRF2, SLC12A1 and CD24, and (c) a DNA polymerase for generating amplified DNA from the cDNA.

In several embodiments, the kit further comprises a filter for capturing vesicles from the urine sample. In several embodiments, the kit further comprises a lysis buffer for liberating RNA from the vesicles. In several embodiments, the kit further comprises an elution buffer for transporting RNA from the lysed vesicles to an analysis vessel. In several embodiments, the kit additionally comprises a microplate configured to receive the RNA. In some embodiments, the microplate comprises oligo(dT) in each well of the plate.

In several embodiments, the kit further comprises a DNA amplification buffer comprising Tris-HCl, magnesium chloride, potassium chloride, and adenine, thymine, guanine, and cytosine nucleotides.

In several embodiments, the kit further comprises at least one fluorescent probe complementary to a region within the amplified DNA of one of PPARGC1A, SMAD1, UMOD, NRF2, SLC12A1 and CD24. In several such embodiments, the kit optionally comprises a means for detecting the fluorescent probe.

In several embodiments, the kits enable the detection of diabetes-induced damage to the nephron. In several embodiments, the diabetes-induced damage to the nephron is correlated with one or more non-molecular indicators of diabetic nephropathy.

In several embodiments, the kit further comprises control DNA indicating expression levels of one of PPARGC1A, SMAD1, UMOD, NRF2, SLC12A1 and CD24 in the absence of diabetic nephropathy.

In several embodiments, the kits are suitable for detecting diabetic nephropathy that is due to Type I or Type II diabetes. In several embodiments, advantageously, the kits are able to detect diabetic nephropathy when associated Type I or Type II diabetes has not yet been diagnosed.

The methods summarized above and set forth in further detail below describe certain actions taken by a practitioner; however, it should be understood that they can also include the instruction of those actions by another party. Thus, actions such as "administering a blood test" include "instructing the administration of a blood test."

### BRIEF DESCRIPTION OF THE FIGURES

Figures 1A-1H depict analysis of mRNA levels from control and diabetic patients.
Figures 2A-2H depict analysis of mRNA levels correlated with blood levels of HbA1c.
Figures 3A-3H depict analysis of mRNA levels correlated with serum creatinine levels.

### DETAILED DESCRIPTION

### General

Interpretation of a patient's symptoms, evaluation of their medical history and performance of a physical exam are typically used to generate an initial diagnosis, which is often corroborated with one or more medical tests. Many diagnostic medical tests are performed on blood extracted from a patient, as obtaining the sample is a relatively non-invasive. Tests may measure concentrations of certain molecules in the sample, which are then compared to normal concentrations (e.g., healthy ranges) or to concentrations measured in a prior sample from the patient. Other tests may also be available to evaluate kidney function, such as measurement of glomerular filtration rate (GFR) or clearance of certain pharmacological marker compounds (e.g., analysis of urine samples over time). Another prognostic marker for kidney function is proteinuria, an elevated level of protein in the urine. Increasing amounts of proteins (such as albumin) in the urine indicate progressively increasing amounts of kidney damage, and associated loss of function. Unfortunately, many diagnostic tests are only sufficiently sensitive to detect measurable increases in a molecule (or molecules) associated with a disease or injury at such a time when significant disease progression or injury has already occurred. For example, in the context of evaluating kidney function, plasma concentrations of molecules such as creatinine or urea (waste substances that should be removed by a functional kidney) often will not be raised above the normal range until a substantial amount (e.g., 40% or greater) of total kidney function is lost.

Some of the assays described above rely on antigen-based detection of a marker or molecule, which can introduce some limitations with respect to sensitivity of the assay. Some assays employ more simplistic chemical reactions (e.g., colorimetric changes) to identify markers from blood or other fluid samples, however, these too may suffer from limitations of sensitivity. Questionable assay accuracy at low assay target concentration ranges significantly limits the ability of these assays to detect early stages of disease of injury that compromise kidney function. Thus, there exists a need for a sensitive, accurate and reproducible diagnostic test for evaluating kidney function that enable early detection and/or diagnosis of compromised kidney function.

### Vesicle-Associated RNA

As discussed in more detail below, several embodiments of the methods disclosed herein are based on the identification of specific nucleic acids that are markers of disease or injury to the kidney. Advantageously, the nucleic acid-based methods provide a higher degree of sensitivity than the alternative assays disclosed above. In several embodiments, the nucleic acids are isolated from cells that are obtained from a blood or urine sample. In other embodiments, the nucleic acids exist extracellularly and are collected in a cell-free preparation. While several embodiments disclosed herein are directed to the isolation of RNA associated with vesicles present in patient urine samples, in several embodiments, RNA (and the associated markers) that are normally found in blood or plasma are isolated from urine samples. In some embodiments, these blood-borne markers are present in the urine due to damage or disease of the kidney that has compromised the normal blood filtering function of the kidney

In several embodiments disclosed herein, there are provided methods for the capture of RNA from a sample of patient body fluid and subsequent analysis of that RNA for disease and/or tissue specific markers. In several embodiments, the method comprises isolated of vesicles associated with RNA from a patient urine sample. In other embodiments, vesicles are obtained from plasma, serum, cerebrospinal fluid, sputum, saliva, mucus, tears etc. Many diagnostic tests are designed around using a small patient fluid sample, and in some embodiments, a small amount (e.g. 15-50 mL of urine) is used. However, several embodiments are particularly advantageous because large volumes of patient urine are readily available.

As described below, in some embodiments, the nucleic acids are vesicle-associated. In some embodiments, the nucleic acids detected are indicative of kidney disease and/or function (e.g., they not normally present in the urine of subject's having normal kidney function). In some embodiments, the detection of the nucleic acids is associated with severity and/or progression of kidney disease or injury (e.g., the nucleic acids are present in the patient urine sample at a greater or lesser concentration as compared to a population of individuals known to have normal kidney function). In some embodiments, urine is collected and nucleic acids are evaluated over time (e.g., to monitor a patient's response to therapy or disease progression).

According to various embodiments, various methods to quantify RNA are used, including Northern blot analysis, RNAse protection assay, PCR, nucleic acid sequence-based amplification, branched-DNA amplification, ELISA, mass spectrometry, CHIP-sequencing, and DNA or RNA microarray analysis.

RNA (and other nucleic acids) are typically within the intracellular environment. However, certain nucleic acids exist extracellularly. For example, in several embodiments, the methods involve collection and analysis of naked extracellular nucleic acids (e.g., naked RNA). This is advantageous in several embodiments because, typically, the extracellular environment that comprises substantial quantities of RNAses leads to rapid degradation of the nucleic acids.

In several embodiments, nucleic acids are associated with extracellular vesicles. In several embodiments, diagnosis and characterization of kidney disease/function is performed by detection and quantification of specific RNA species from RNA-containing vesicles isolated from patient samples (e.g., urine). In one embodiment, such vesicles are trapped on a filter, thereby allowing RNA extraction from the vesicles. In additional embodiments, centrifugation is used to collect the vesicles.

Nucleic acids can be associated with one or more different types of membrane particles (ranging in size from 50-80 nm), exosomes (ranging in size from 50-100 nm), exosome-like vesicles (ranging in size from 20-50 nm), and microvesicles (ranging in size from 100-1000nm). In several embodiments, these vesicles are isolated and/or concentrated, thereby preserving vesicle associated RNA despite the high RNAse extracellular environment. In several embodiments, the sensitivity of methods disclosed here is improved (vis-à-vis isolation of nucleic acids from tissues and/or collection of naked nucleic acids) based on the use of the vesicle-associated RNA.

A variety of methods can be used, according to the embodiments disclosed herein, to efficiently capture and preserve vesicle associated RNA. In several embodiments, centrifugation on a density gradient to fractionate the non-cellular portion of the sample is performed. In some embodiments, density centrifugation is optionally followed by high speed centrifugation to cause vesicle sedimentation or pelleting. As such approaches may be time consuming and may require expensive and specialized equipment in several embodiments, low speed centrifugation can be employed to collect vesicles.

In several embodiments, filtration (alone or in combination with centrifugation) is used to capture vesicles of different sizes. In some embodiments, differential capture of vesicles is made based on the surface expression of protein markers. For example, a filter may be designed to be reactive to a specific surface marker (e.g., filter coupled to an antibody) or specific types of vesicles or vesicles of different origin.

In some embodiments, the markers are unique vesicle proteins or peptides. In some disease states, the markers may also comprise certain modifications, which
maybe used to isolate particular vesicles. Modification may include, but are not limited to addition of lipids, carbohydrates, and other molecules such as acylated, formylated, lipoylated, myristolylated, palmitoylated, alkylated, methylated, isoprenylated, prenylated, amidated, glycosylated, hydroxylated, iodinated, adenylated, phosphorylated, sulfated, and selenoylated, ubiquitinated. The vesicle markers may comprise non-proteins such as lipids, carbohydrates, nucleic acids, RNA, DNA, etc.

The specific capture of vesicles based on their surface markers also enables a "dip stick" format where each different type of vesicle is captured by dipping probes coated with different capture molecules (e.g., antibodies with different specificities) into a patient urine sample.

### Kidney Structure, Function, and Disease

The anatomy of the kidney is divided into two main tissue types, the renal cortex (the superficial area) and the renal medulla (the more interior area). Nephrons, the functional unit of the kidney, span the cortex and medulla. The initial filtering portion of a nephron is the renal corpuscle, located in the cortex, which leads to a renal tubule that passes from the cortex deep into the medulla.

A portion of the renal corpuscle, the glomerulus, performs the first step in filtering blood to form urine. The unique anatomy of the kidney leads to a high back-pressure in the glomerulus (due to the glomerulus draining into an arteriole rather than a venule). The back-pressure aids in the filtration process, but also has the potential to lead to kidney damage in certain disease states. Diabetes is characterized by elevated levels of glucose, a relatively large solute, in the blood. When diabetes is uncontrolled, the excess glucose can lead to physical damage to the glomerulus, which is exacerbated over time, as the initial damage to the glomerulus allows increased blood flow speed through the glomerulus, which results in the potential for further glucose-derived damage to the glomerulus.

After passing through the glomerulus, filtrate passes through the proximal tubule, the loop of Henle, the distal convoluted tubule, and the collecting duct. In sum, these anatomical structures function to generate a concentration gradient from the cortex to the medulla, which allows for the reabsorption of water from the filtrate, which creates concentrated urine for excretion.

Common clinical conditions involving the kidney include nephritic damage (either to the glomerulus specifically or the kidney generally), renal cysts, acute kidney injury, chronic kidney disease, urinary tract infection, nephrolithiasis (kidney stones), and urinary tract obstruction. Various cancers of the kidney exist, including, but not limited to, renal cell carcinoma, Wilms tumor, and renal cell carcinoma.

Several embodiments described herein are advantageous because markers associated with kidney function and/or disease can be rapidly assessed in a high through put protocol. Several embodiments are used to diagnose and/or monitor various kidney diseases (or loss of function related thereto), including, but not limited to chronic kidney disease, acute renal failure, diabetic nephropathy, glomerulonephritis, glomerulosclerosis, focal segmental glomerulosclerosis, membranous nephropathy, minimal change disease, and kidney disease secondary to other diseases such as atherosclerosis, hypertension, cardiovascular diseases, obesity, hypercholesterolemia, diabetes (e.g., diabetic nephropathy), collagen diseases, as well as kidney damage caused by pharmaceuticals or other compounds.

Damage to the kidney vasculature, in particular the endothelium of renal blood vessels, may be detected by evaluation of kidney endothelial cell-specific mRNA. The markers may be related to blood homeostasis such as endothelia cell marker von Willebrand factor (VWF), thrombin, factor VIII, plasmin, and fibrin. Von Willebrand factor is a plasma glycoprotein that is a mediator of platelet adhesion, as such it is released when the endothelium is damaged. VWF is involved in platelet aggregation and thrombus formation. In some embodiments, the markers may be kidney markers, such as, for example, Tamm-Horsfall glycoprotein (THP) also known as uromodulin, renin, solute carrier transporters (including, among others, SLC12A1, SLC22A6, SLC22A8, and SLC22A12), uromodulin associated kidney disease marker (UMOD), osteopontin (SPP1), and albumin (ALB), kidney fibrosis markers, such as matrix metallopeptidase 1 (MMP1) and matrix metallopeptidase 3 (MMP3), glomerular markers (e.g., glomerulus-specific (podocine (PDCN)), proximal tubule markers (e.g., proximal tubule-specific (uromodulin (UMOD)), albumin (ALB), Na/K/Cl transporter (SLC12A1)), distal tubule-specific markers (e.g., aquaporin 9 (AQP9)), as kidney-diabetes related markers including but not limited to peroxisome proliferator-activated receptor gamma, coactivator 1 α and β (PPARGC1A and B), nuclear respiratory factor 1 and 2 (NRF1 and 2), estrogen-related receptor α (ESRRA), annexin A5 (ANXA5), protein kinase, AMP-activated, α₁ and α□₂ catalytic subunit (PRKAA1 and 2), uncoupling protein 1 and 2 (UCP1 and 2), low density lipoprotein receptor-related protein 2 (LRP2), CD24, secreted phosphoprotein 1 (SPP1), α2-HS-glycoprotein (AHSG), SMAD family member 1 (SMAD1)) and the like.

Several embodiments of the methods disclosed herein provide unexpected advantages over existing diagnostic and monitoring methods. For example, some diagnostic tests for kidney disease require a kidney biopsy, which is typically performed via puncture of the organ with a needle. The biopsy technique has the associated risks such as uncontrolled bleeding and infection. The methods described herein provide an opportunity to non-invasively identify RNA which indicates loss of kidney function due to diabetes (or other sources of kidney damage). Several embodiments thus unexpectedly enable remote sampling and assessment of the kidney without the associated increase in patient risk.

In addition to directly detecting direct kidney disease or injury, several embodiments of the methods disclosed herein are particularly advantageous because they are used to correlate a loss of kidney function (or symptoms thereof) with other diseases that are not kidney specific, but secondarily impact the kidney, for example, diabetes mellitus

As discussed above, elevate blood glucose levels can lead to kidney damage and eventual reduction in kidney function. In some cases, diabetes mellitus leads to development of or is associated with one or more types of cardiovascular disease, which can further exacerbate kidney damage. In a healthy individual with normal functioning metabolism, insulin is produced by beta cells of the pancreas. The subsequent insulin release enables cells to absorb glucose. In contrast, in a diseased state the cells do not absorb glucose and it accumulates in the blood. This may lead to complications and/or damage to the kidney, as well as complications such as cardiovascular disease (coronary artery disease, peripheral vascular disease, and hypertension). Depending on the type of diabetes, a patient with diabetes either does not produce enough insulin or their cells do not properly respond to the insulin that their body does produce. In many cases, pre-diabetic individuals and/or those with diabetes live with early symptoms that are dismissed as being associated with other aspects of their lives or health. For example, post-prandial nausea may be ignored as heartburn, when in fact, the symptom is attributable to elevated blood glucose levels. Ignoring such symptoms over time can lead to, among other symptoms, excessive kidney damage prior to actual diagnosis. The methods disclosed herein can be implemented in routine physical examinations to detect early markers of kidney damage due to diabetes before the symptoms become so severe that irreversible kidney damage is already sustained.

House-keeping gene products or constitutively expressed gene products, or markers of basal cellular function are used as markers or controls against which markers of diabetic nephropathy may be compared. House-keeping genes include, but are not limited to, glyceraldehyde 3-phosphate dehydrogenase, β actin (ACTB), and β2 microglobulin (B2M). Other house-keeping genes known in the art are used in other embodiments.

The functional status of a patient's kidneys may be monitored over time, thereby allowing for the patient's kidney function be quantified at multiple time points. This data allows for tracking of the disease progress which in turn,
enables a medical professional to advise the patient with respect to what additional therapies and/or lifestyle changes might be required of a patient having a kidney disease, such as diabetic nephropathy.

A first sample of urine may be collected from a patient and the level of vesicle or particle associated RNA for a specific gene or genes is determined. A subsequent sample (or samples) is collected from the patient and the level of specific RNA is determined. Any changes in kidney of the patient may thus be determined by comparing the first sample RNA level with the second sample RNA level or by comparing the samples to a control or standard. Medication may have been administered to the patient before or after the collection of the first and/or second patient sample. As disclosed herein, the medication may be a drug, nutritional supplement, vitamin, immunosuppressant, anti-inflammatory drug, anesthetic or analgesic, stem cell, graft, or kidney transplant. The monitoring may relate to a change in nutrition such as a reduction in caloric intake, or increased hydration, or change in exercise routine, or a change in sleeping pattern of the patient.

### Methodology

Free extracellular RNA is quickly degraded by nucleases, making it a potentially poor diagnostic marker. As described above, some extracellular RNA is associated with particles or vesicles that can be found in urine. This vesicle associated RNA, which includes mRNA, is protected from the degradation processes in the urine. Microvesicles are shed from most cell types and consist of fragments of plasma membrane. Microvesicles contain RNA, mRNA, microRNA, and proteins and mirror the composition of the cell from which they are shed. Exosomes are small microvesicles secreted by a wide range of mammalian cells and are secreted under normal and pathological conditions. These vesicles contain certain proteins and RNA including mRNA and microRNA. Exosomes can also be released into urine by the kidneys and their detection may serve as a diagnostic tool, as described in several embodiments herein. In addition to urine, exosome-like vesicles may also be found in many body fluids such as blood, ascites and amniotic fluid, among others. Several embodiments evaluate nucleic acids such as small interfering RNA (siRNA), tRNA, and small activating RNA (saRNA), among others.

In several embodiments the RNA isolated from vesicles from the urine of a patient with diabetic nephropathy is used as a template to make complementary DNA (cDNA). In several embodiments, cDNA is amplified using the polymerase chain reaction (PCR). In other embodiments, amplification of nucleic acid and RNA may also be achieved by any suitable amplification technique such as nucleic acid based amplification (NASBA) or primer-dependent continuous amplification of nucleic acid, or ligase chain reaction. Other methods may also be used to quantify the nucleic acids, such as for example, including Northern blot analysis, RNAse protection assay, PCR, nucleic acid sequence-based amplification, branched-DNA amplification, ELISA, mass spectrometry, CHIP-sequencing, and DNA or RNA microarray analysis.

In several embodiments, diabetic nephropathy induces the expression of one or more marker. In several embodiments, the increased expression is measured by the amount of mRNA encoding said markers (in other embodiments, DNA or protein are used to measure expression levels). In some embodiments urine is collected from a patient and directly evaluated. In some embodiments, vesicles are concentrated, for example by use of filtration or centrifugation. Isolated vesicles are then incubated with lysis buffer to release the RNA from the vesicles, the RNA then serving as a template for cDNA which is quantified with methods such as quantitative PCR (or other appropriate amplification or quantification technique). In several embodiments, the level of specific marker RNA from patient vesicles is compared with a desired control such as, for example, RNA levels from a healthy patient population, or the RNA level from an earlier time point from the same patient or a control gene from the same patient.

In several embodiments, the disclosed methods allow the detection of the presence or absence of diabetic nephropathy by measuring the levels of mRNA encoding one or more markers related to diabetic nephropathy. As disclosed herein, the disclosed methods allow the assessment of the progression (or regression) of diabetic nephropathy by measuring the levels of mRNA encoding one or more markers related to diabetic nephropathy. To determine these mRNA levels, mRNA-containing vesicles may be isolated from plasma using a device for isolating and amplifying mRNA. Embodiments of this device are described in more detail in United States Patent Nos.: 7,745,180, 7,939,300, 7,968,288, 7,981,608, 8,076,105, 8,101,344.

Described is a multi-well plate that contains a plurality of sample-delivery wells, a vesicle-capturing filter underneath the wells, and an mRNA capture zone underneath the filter which contains immobilized oligo(dT).

The device may also contain a vacuum box adapted to receive the filter plate to create a seal between the plate and the box, such that when vacuum pressure is applied, the urine is drawn from the sample-delivery wells across the vesicle-capturing filter, thereby capturing the vesicles and allowing non-vesicle urine components to be removed by washing the filters. Other means of drawing the urine samples through the sample wells and through across the vesicle-capturing filter, such as centrifugation or positive pressure, may be used. Vesicles may be captured on a plurality of filter membranes that are layered together. The captured vesicles may then be lysed with a lysis buffer, thereby releasing mRNA from the captured vesicles. The mRNA is then hybridized to the oligo(dT)-immobilized in the mRNA capture zone. Further detail regarding the composition of lysis buffers that may be used in several embodiments can be found in United States Patent No.: 8,101,344.
cDNA may be synthesized from oligo(dT)-immobilized mRNA. The cDNA may then be amplified using real time PCR with primers specifically designed for amplification of disease-associated markers. Primers that are used in such embodiments are shown in Table 1. Further details about the PCR reactions used in some embodiments are also found in United States Patent Application No.: 8,101,344.

**Table 1: Primer Sequences for RT-PCR Amplification**

| **Target** | **FWD Sequence (5'-3')** | **REV Sequence (3'-5')** |
|---|---|---|
| β-Actin | CCTGGCACCCAGCACAAT (SEQ ID No. 1) | GCCGATCCACACGGAGTACT (SEQ ID No. 2) |
| β-2 microglobulin (B2M) | TGACTTTGTCACAGCCCAAGATA (SEQ ID No. 3) | AATGCGGCATCTTCAAACCT (SEQ ID No. 4) |
| PDCN (glomerulus specific podocin) | AGGATGGCAG CTGAGATTCT GT (SEQ ID No. 5) | AGAGACTGAA GGGTGTGGAG GTAT (SEQ ID No. 6) |
| UMOD (uromodulin) | CCTGAACTTG GGTCCCATCA (SEQ ID No. 7) | GCCCCAAGCT GCTAAAAGC (SEQ ID No. 8) |
| ALB (albumin) | TGCAAGGCTGACGATAAGGA (SEQ ID No. 9) | GTAGGCTGAGATGCTTTTAAATGTGA (SEQ ID No. 10) |
| SLC12A1 (Na⁺/K⁺/Cl⁻ transporter) | ACTCCAGAGCTGCTAATCTCATTGT (SEQ ID No. 11) | AACTAGTAAGACAGGTGGGAGGTTCT (SEQ ID No. 12) |
| AQP9 (distal tubule specific aquaporin 9) | AAACAACTTCTGGTGGATTCCTGTA (SEQ ID No. 13) | GCTCTGGATGGTGGATTTCAA (SEQ ID No. 14) |
| PPARGC1A (peroxisome proliferator-activeted receptor gamma, coactivator 1 α) | GCTCTTGAAAATGGATACACTTTGC (SEQ ID No. 15) | TCTGAGTTTGAATCTAGGTCTGCATAG (SEQ ID No. 16) |
| PPARGC1B (peroxisome proliferator-activated receptor gamma, coactivator 1 β) | CCCTTCTCCTGTTCCTTTGGA (SEQ ID No. 17) | CCTTTGCAGGACGCCTTCT (SEQ ID No. 18) |
| NRF1 (nuclear respiratory factor 1) | CCAGATCCCTGTGAGCATGTAC (SEQ ID No. 19) | TGACTGCGCTGTCTGATATCCT (SEQ ID No. 20) |
| NRF2 (nuclear respiratory factor 2) | CATGCTACGTGATGAAGATGGAA (SEQ ID No. 21) | AACAAGGAAAACATTGCCATCTC (SEQ ID No. 22) |
| ESRRA (estrogen-related receptor α) | AAAGTGCTGGCCCATTTCTATG (SEQ ID No. 23) | TCTCCAAGAACAGCTTGTGCAT (SEQ ID No. 24) |
| ANXA5 (annexin 5) | TGGTTTCCAGGAGTGAGATTGA (SEQ ID No. 25) | TGGAATAAAGAGAGGTGGCAAAA (SEQ ID No. 26) |
| PRKAA1 (AMP-activated, α1 catalytic subunit) | TCAGATGCTGAGGCTCAAGGA (SEQ ID No. 27) | TGTGTGACTTCCAGGTCTTGGA (SEQ ID No. 28) |
| PRKAA2 (AMP-activated, α2 catalytic subunit) | CTGCAGAGAGCCA TTCACTTTCT (SEQ ID No. 29) | GGTGAAACTGAAGACAATGTGCTT (SEQ ID No. 30) |
| UCP1 (uncoupling protein 1) | GGACCAACGGCTTTCTTCAA (SEQ ID No. 31) | CATAATGACGTTCCAGGATCCA (SEQ ID No. 32) |
| UCP2 (uncoupling protein 2) | GCTTGGGTTCCTGGAACGT (SEQ ID No. 33) | AGCCATGAGGGCTCGTTTC (SEQ ID No. 34) |
| LRP2 (low density lipoprotein receptor-related protein 2) | GCACAGATGG AGAACGAGCA A (SEQ ID No. 35) | AGCAGGGAGC GAAGGTGAT (SEQ ID No. 36) |
| CD24 | GACACTCCCC GAAGTCTTTT GT (SEQ ID No. 37) | TCATCAAGAC TACTGTGGCCATATTAG (SEQ ID No. 38) |
| SPP1 (secreted phosphoprotein 1) | AGCCAATGAT GAGAGCAATG AG (SEQ ID No. 39) | TGGAATTCAC GGCTGACTTT G (SEQ ID No. 40) |
| AHSG (α2-HS-glycoprotein) | CATGGGTGTGGTCTCATTGG (SEQ ID No. 41) | CAACACTAGGCTGCACCACTGT (SEQ ID No. 42) |
| SMAD1 (SMAD family member 1) | CTGCTATTCT GAAATTGCCT ACATG (SEQ ID No. 43) | ACTGTAAACT CCGTAAAAAC TGCTTATTAA (SEQ ID No. 44) |

After the completion of the PCR reaction, the mRNA (as represented by the amount of PCR-amplified cDNA detected) for one or more markers is quantified.

Quantification may be calculated by comparing the amount of mRNA encoding a disease marker to a reference value. The reference value may be the amount of mRNA found in healthy non-diseased patients.
The reference value may be the expression level of a house-keeping gene.

Beta-actin, or other appropriate house-keeping gene may be used as the reference value. Numerous other house-keeping genes that are well known in the art may also be used as a reference value. A house-keeping gene may be used as a correction factor, such that the ultimate comparison is the expression level of marker from a diseased patient as compared to the same marker from a non-diseased (control) sample.

The house-keeping gene may be a tissue specific gene or marker, such as those discussed above. The reference value may be zero, such that the quantification of the markers is represented by an absolute number.
A ratio comparing the expression of one or more markers from a diseased patient to one or more other markers from a non-diseased person may be made.

Expression of markers related to diabetic nephropathy may be measured before and/or after administration of a drug (or other therapy) to a patient. The expression profiles may be used to predict the efficacy of a drug compound (e.g. in treating diabetic nephropathy) or to monitor side effects of the drug compound (e.g., impact on kidney function). The drug monitored may have been administered to treat one or more of chronic kidney disease, acute renal failure, diabetic nephropathy, glomerulonephritis, glomerulosclerosis, focal segmental glomerulosclerosis, membranous nephropathy, minimal change disease, atherosclerosis, hypertension, cardiovascular diseases, obesity, hypercholesterolemia, diabetes, collagen diseases, cancer drug, infections, and/or immunosuppressive diseases.
A drug compound may induce the expression of a distinctive mRNA profile. Likewise,
a drug may inhibit expression of one or more markers. As disclosed herein,
the efficacy of drug treatment can be monitored by the disappearance (or
reduced expression) of markers associated with a particular disease state. As also disclosed herein, the methods disclosed herein evaluate a change in diet, lifestyle, or other non-traditional (e.g., non-drug) therapy on the function of a diabetic subject's kidneys.

As disclosed herein, the analyses described herein are applicable to human patients, while it is described that the methods may also be applicable to animals (e.g., veterinary diagnoses).

### EXAMPLES

Specific embodiments will be described with reference to the following examples which should be regarded in an illustrative rather than a restrictive sense.

### Example 1- Identification of Biomarkers Associated With Diabetic Nephropathy

For many diabetic patients, early diagnosis of kidney problems followed by appropriate treatment or strict blood glucose control is only way to prevent end-stage kidney disease. As discussed above, however, kidney function tests are relatively limited and often insufficiently sensitive to detect early signs of kidney problems. The invasive nature of kidney precludes its use as routine diagnostic test.

Given ready access to potentially large quantities of patient urine samples, several embodiments of the methods disclosed herein employ urine as a diagnostic sample. Many current diagnostic tests measure solutes excreted in urine, or measure urine production rate, in order to evaluate kidney function, or loss thereof. However, several embodiments of the methods disclosed herein exploit the presence of nucleic acid-containing vesicles present in the urine make a sensitive and specific diagnostic analysis of kidney function based on isolation and amplification of kidney specific markers.

### Methods

### Samples

Urine samples were obtained from healthy donors (n=23) and diabetic nephropathy patients (n=23) at the hospital of University of California San Diego.

### Exosomal mRNA analysis.

Each urine sample was centrifuged at 1,000 x g for 15 minutes, and 10 mL of the resulting supernatant was applied (by vacuum) to a 96-well exosome-capture filterplate. The filterplate was then centrifuged at 2,000 x g for an additional 5 minutes. In each well, 60 µL of Lysis buffer containing a cocktail of antisense primers were added, and incubated at 55°C for 10 minutes. The resultant lysate was transferred from the filterplate to an oligo(dT)-immobilized 96-well microplate by centrifugation at 2,000 x g for 5 minutes. cDNA was directly synthesized in the same oligo(dT)-immobilized 96-well microplate by adding dNTPs (final concentration of 5 mM), MMLV reverse transcriptase (final concentration of 2.7 U/mL), and RNasin (final concentration of 0.13 U/mL) (Invitrogen, Carlsbad, CA) and incubation at 37°C for 2 hours. cDNA was subsequently used in real time SYBR green PCR using iTaqSYBR master mix (BioRad, Hercules, CA) by established methods (see e.g., Mitsuhashi M, J Immunol Methods. 363:95-100, 2010
). PCR conditions were 50 cycles of annealing at 65°C for 1 minute followed by denaturization at 95°C for 30 seconds using a PRISM 7900 (Applied Biosystems (ABI), Foster City, CA). The results were expressed as the cycle threshold (Ct) using the analytical software (SDS, ABI). Ct = 32 was considered as the baseline.

### Targeting mRNAs

A total of 23 mRNAs were quantified, and included: 2 control genes (β-actin (ACTB) and β2 microglobulin (B2M)), glomerulus-specific (podocine (PDCN)), proximal tubules-specific (uromodulin (UMOD), albumin (ALB), and Na/K/Cl transporter (SLC12A1)), distal tubules-specific (aquaporin 9 (AQP9)), as well as kidney-diabetes related miscellaneous mRNAs (peroxisome proliferator-activated receptor gamma, coactivator 1 α and β (PPARGC1A and B), nuclear respiratory factor 1 and 2 (NRF1 and 2), estrogen-related receptor α (ESRRA), annexin A5 (ANXA5), protein kinase, AMP-activated, α1 and α2 catalytic subunit (PRKAA1 and 2), uncoupling protein 1 and 2 (UCP1 and 2), low density lipoprotein receptor-related protein 2 (LRP2), CD24, secreted phosphoprotein 1 (SPP1), α2-HS-glycoprotein (AHSG), and SMAD family member 1 (SMAD1)).

### Results

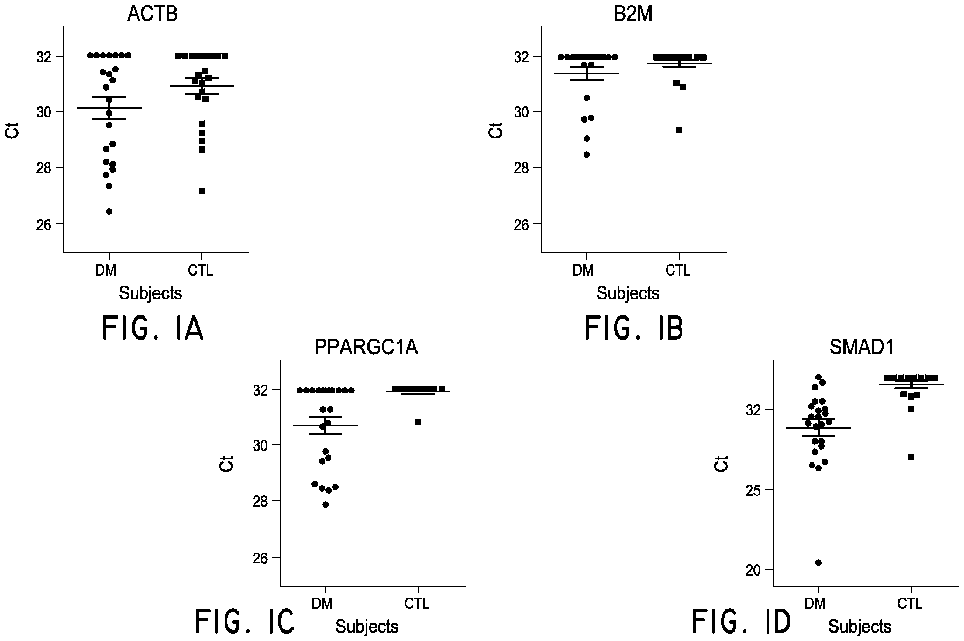

Expression levels of various exosomal mRNA from either diabetic (DM) or normal (CTL) patients were compared (Figures 1A-1H). As shown in Fig. 1A/1B, expression of the control genes (β-actin, ACTB) and B2M did not differ based on presence or absence of diabetes. In contrast, significant increases in expression of PPARGC1A, SMAD1, UMOD, NRF2, SLC12A1 and CD24 were detected in DM patients. These data indicate that these mRNAs (as well as others that are increased in response to diabetes-induced, or other type, of kidney damage) are correlated with the presence of diabetes. Their upregulation indicate their possible utility as biomarkers associated with the disease and its related loss of kidney function.

In order to characterize the severity of the diabetic condition in each DM patient, exosome mRNA expression data was correlated with results of HbA1c testing. The HbAlc test evaluates the blood glucose levels in a diabetic patient over time. Clinicians generally view an HbAlc of 5.6% or less is normal. Ranges of 5.7% to 6.4% are associated with pre-diabetes, while levels of 6.5% or higher leads to a diagnosis of diabetes. Above 6.5%, increased HbAlc levels are correlated with increasingly severe dysregulation of blood glucose, and thus increased risk for diabetic nephropathy.

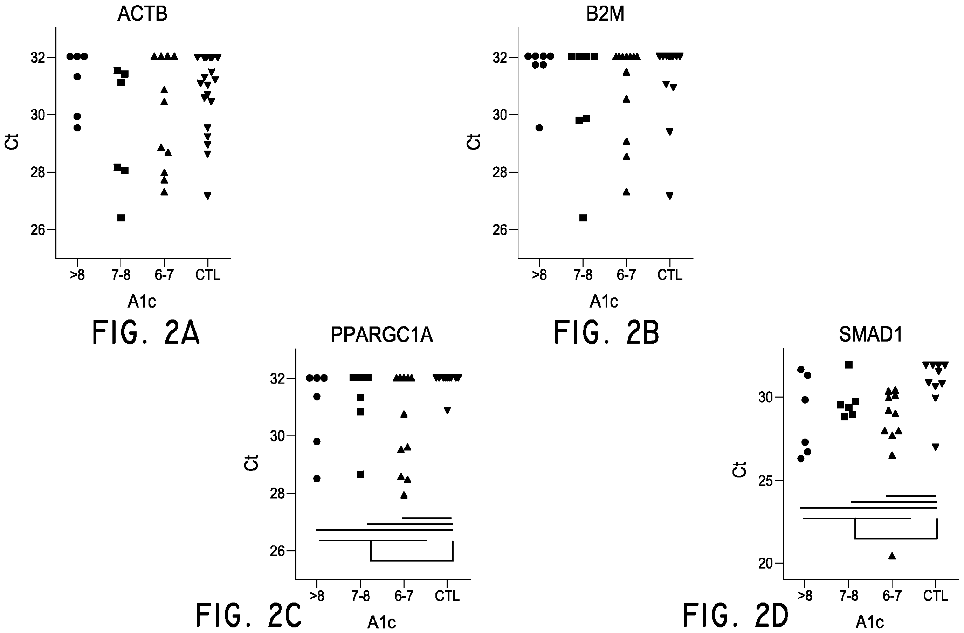

As shown in Figures 2A-2H, even those DM patients with only a slight increase of HbAlc (6-7%) demonstrated significant increased expression (versus healthy controls) of 5 genes (PPARGC1A, SMAD1, UMOD, NRF2, SLC12A1, not CD24). Given the modest increase in HbA1c levels, these data suggest that these mRNAs are sensitive biomarkers of DM.

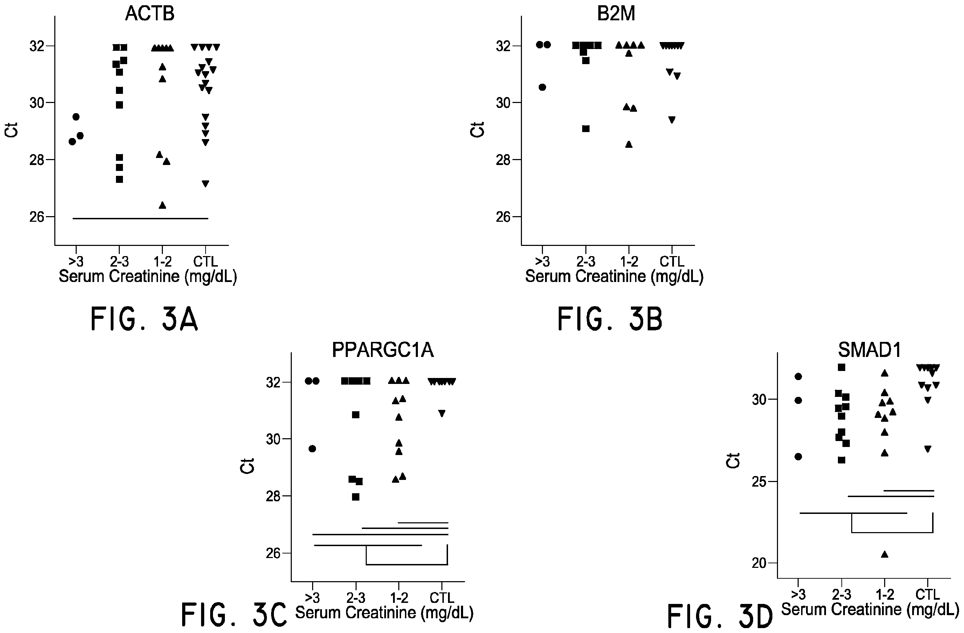

To further evaluate candidate biomarkers of kidney damage, urine exosome mRNAs were then compared with the levels of serum creatinine. Creatine phosphate is metabolized by the muscles to produce energy and creatinine is produced as a waste product. Creatinine is carried by the blood to the kidneys where it is excreted in the urine. Generally, since creatinine production is linked to muscle mass, which varies little from day to day, so creatinine level should remain relatively constant if kidneys are functioning properly. Increased creatinine levels are indicative of reduced filtration, which is a hallmark of diabetic-induced damage to the nephrons. As shown in Figures 3A-3H, DM patients with only a slight increase in serum creatinine (1-2 mg/dL) still showed significant differences in gene expression (against healthy controls) in 4 genes (PPARGC1A, SMAD1, UMOD, SLC12A1). As with the HbAlc data, these results suggest that mRNAs are sensitive biomarkers of kidney damage. Thus, these markers, or others that are elevated in the early stages of kidney damage are used in several embodiments to identify kidney damage at its earliest stages (before other analytical methods would detect severe damage and prior to detectable symptoms).

### Discussion

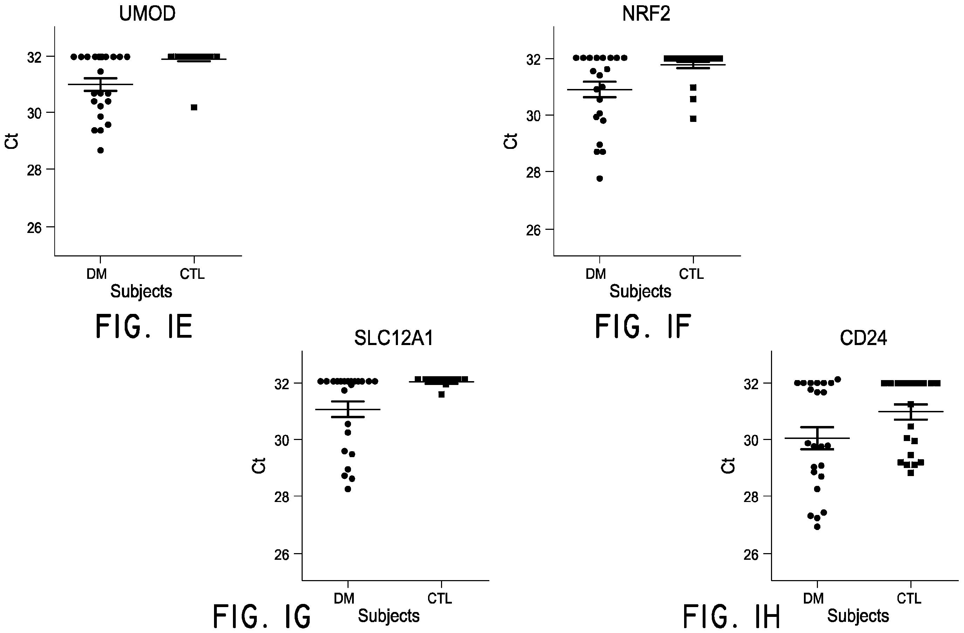

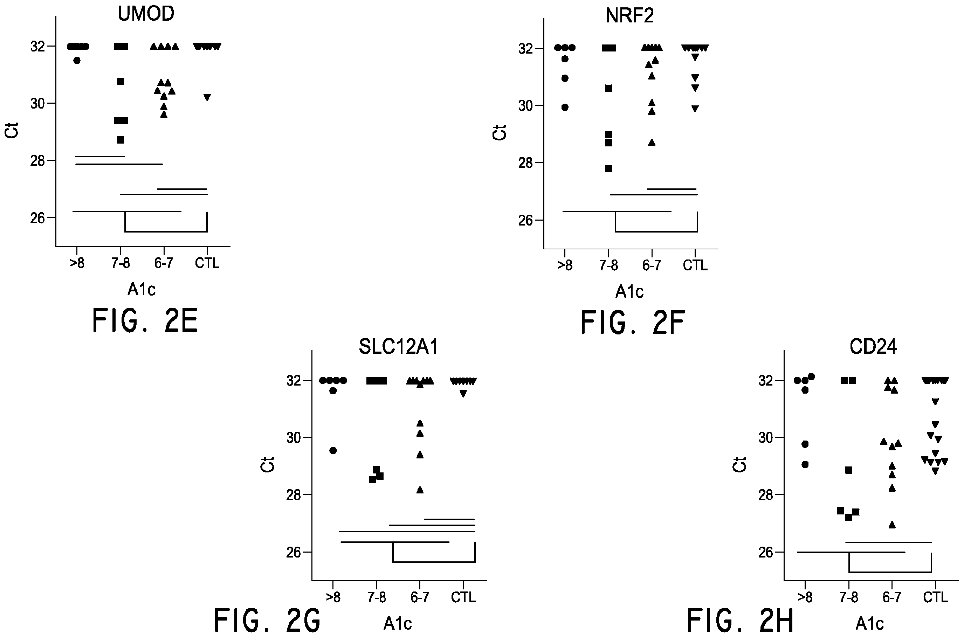

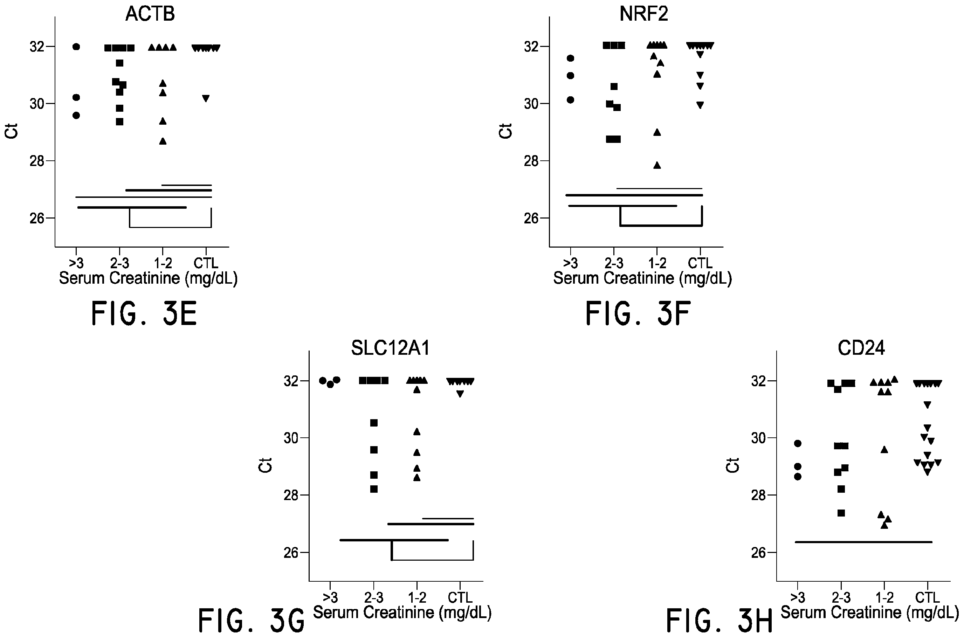

The data presented above demonstrate that certain mRNA markers can be isolated from patient urine samples, processed, quantified, and correlated to diabetic nephropathy. These data also indicate that certain markers correlated with established diagnostic markers used to identify patients suffering from diabetic nephropathy. As shown in Figure 1, clear differences in expression levels could be in certain markers when normal subjects were evaluated in comparison to subjects with diabetes. Figures 2 and 3 demonstrate that several markers are indicative of the severity of the damaged to the kidney due to diabetes. As shown, the mRNA markers are correlated with traditional diagnostic endpoints. Advantageously, however, the methods disclosed herein are non-invasive (whereas traditional tests require blood draws) can easily and routinely be repeated, and are highly sensitive. This sensitivity, as discussed above, is particularly advantageous as it allows the early detection of diabetic nephropathy, in many cases prior to the ability of a patient or doctor to identify symptoms. As such, in several embodiments, the claimed methods allow preventative action to take place (e.g., lifestyle change, more robust therapy to control the diabetes etc.) and thus prevent disease progression, or at least reduce the severity of the progression. In addition, the high degree of correlation with currently used clinical markers, allows the methods disclosed herein to be used to identify additional genetic markers of diabetic nephropathy, including those that are indicative of the severity of the disease.

It is disclosed herein that various combinations or subcombinations of the specific features and aspects of the embodiments disclosed .above may be made and still fall within one or more of the inventions. Further, it is disclosed that the disclosure herein of any particular feature, aspect, method, property, characteristic, quality, attribute, element, or the like in connection with an embodiment can be used in all other embodiments set forth herein. Accordingly, it
is disclosed that various features and aspects of the disclosed embodiments can be combined with or substituted for one another in order to form varying modes of the disclosed inventions. Thus, it is disclosed that the scope of the present inventions herein disclosed should not be limited by the particular disclosed embodiments described above. Moreover, while the invention is susceptible to various modifications, and alternative forms, specific examples thereof have been shown in the drawings and are herein described in detail.

The methods disclosed herein include certain actions taken by a practitioner, however, they can also include any third-party instruction of those actions, either expressly or by implication. For example, actions such as "administering a blood test" include "instructing the administration of a blood test." The ranges disclosed herein also encompass any and all overlap, sub-ranges, and combinations thereof. Language such as "up to," "at least," "greater than," "less than," "between," and the like includes the number recited. Numbers preceded by a term such as "about" or "approximately" include the recited numbers. For example, "about 3 mm" includes "3 mm."

## Claims

1. A method for identifying a subject currently affected by diabetic nephropathy due to Type I or Type II diabetes, comprising:
isolating vesicles that are associated with RNA from a sample of urine that has been obtained from a subject, wherein said sample comprises vesicles that are associated with RNA;
lysing said vesicles to release said vesicle-associated RNA, wherein said vesicle-associated RNA comprises an RNA associated with diabetes-induced damage to the nephron,
wherein said RNA associated with diabetes-induced damage to the nephron is selected from the group consisting of PPARGC1A, SMAD1, NRF2, UMOD, SLC12A1 and CD24;
quantifying said RNA associated with diabetes-induced damage to the nephron by a method comprising:
(i) contacting said RNA from said sample with a reverse transcriptase to generate complementary DNA (cDNA), and
(ii) contacting said cDNA with sense and antisense primers that are specific for one of PPARGC1A, SMAD1, NRF2, UMOD, SLC12A1 and CD24 and a DNA polymerase to generate amplified DNA;
comparing the amount of quantified RNA associated with diabetes-induced damage to the nephron from said sample to a quantity of a corresponding RNA from individuals having normal kidney function; and
identifying a subject currently affected by diabetic nephropathy due to Type I or Type II diabetes when there is a difference between the quantity of said RNA associated with diabetes-induced damage to the nephron in said subject and said quantity of the RNA in individuals with normal kidney function.

2. The method of Claim 1, wherein the difference in the quantity of RNA associated with diabetes-induced damage to the nephron is correlated with one or more non-molecular indicators of diabetic nephropathy.

3. The method according to Claim 1 or 2, wherein said RNA associated with diabetes-induced damage to the nephron is selected from the group consisting of PPARGCIA, SMAD1, UMOD and SLC12A1.

4. The method according to any one of Claims 1 to 3, wherein isolating the vesicles from said sample comprises filtering the urine, wherein said filtration traps said vesicles on the filter.

5. The method of Claim 4, wherein said lysing is performed while said vesicles are trapped on said filter.

6. The method according to any one of Claims 1 to 5, further comprising centrifuging said sample to remove cellular debris, wherein said centrifugation is performed prior to isolating the vesicles.

7. The method of Claim 6, wherein concentrating the vesicles further comprises filtering the supernatant of said centrifuged urine.

8. The method according to any one of Claims 1 to 7, wherein said Type I or Type II diabetes has not yet been diagnosed.

9. The method according to any one of Claims 1 to 8, wherein said RNA associated with diabetes-induced damage to the nephron comprises poly(A)+ RNA.

10. A method according to any one of the preceding Claims, wherein the method is used to screen a plurality of subjects to determine their likelihood of developing diabetic nephropathy due to Type I or Type II diabetes and/or to detect early stage diabetic nephropathy due to Type I or Type II diabetes.

11. A method for identifying a subject affected by diabetic nephropathy due to Type I or Type II diabetes, comprising:
isolating vesicles that are associated with RNA from a sample of urine that has been obtained from a subject, wherein said sample comprises vesicles that are associated with RNA;
lysing said vesicles to release said vesicle-associated RNA, wherein said vesicle-associated RNA comprises an RNA associated with diabetes-induced damage to the nephron,
wherein said RNA associated with diabetes-induced damage to the nephron is selected from the group consisting of PPARGC1A, SMAD1, NRF2, UMOD, SLC12A1 and CD24;
quantifying said RNA associated with diabetes-induced damage to the nephron such as by using a method selected from the group consisting of RNA sequencing, RNA microarray, nucleic acid sequence-based amplification, northern blotting, RNase protection assay and branched-DNA amplification; and
comparing the amount of said RNA associated with diabetes-induced damage to the nephron from said subject to the quantity of a corresponding RNA from individuals having normal kidney function, wherein a difference in the quantity of said RNA associated with diabetes-induced damage to the nephron between said subject and said individuals indicates the subject is affected by diabetic nephropathy due to Type I or Type II diabetes.

12. Use of a kit for detection of early stage diabetic nephropathy, comprising:
(a) a reverse transcriptase enzyme for generating complementary DNA (cDNA) from RNA isolated from a urine sample of a subject being evaluated for their diabetic nephropathy status;
(b) at least one pair of sense and antisense primers specific for one of PPARGC1A, UMOD, NRF2, SLC12A1 and CD24; and
(c) a DNA polymerase for generating amplified DNA from the cDNA.

13. The use of the kit of Claim 12, wherein said kit further comprises a filter for capturing vesicles from said urine sample.

14. The use of the kit of Claim 13, wherein said kit further comprises a lysis buffer for liberating RNA from said vesicles.

15. The use of the kit of Claim 14, wherein said kit further comprises an elution buffer for transporting RNA from said lysed vesicles to an analysis vessel.

16. The use of the kit according to any one of Claims 12 to 15, wherein said kit further comprises a microplate configured to receive said RNA.

17. The use of the kit of Claim 16, wherein said microplate comprises oligo(dT) in each well of the plate.

18. The use of the kit according to any one of Claims 12 to 17, wherein said kit further comprises a DNA amplification buffer comprising Tris-HCl, magnesium chlorides, potassium chloride, and adenine, thymine, guanine, and cytosine nucleotides.

19. The use of the kit according to any one of Claims 12 to 18, wherein said kit further comprises at least one fluorescent probe complementary to a region within the amplified DNA of one of PPARGC1A, SMAD1, UMOD, NRF2, SLC12A1 and CD24.

20. The use of the kit of Claim 19, wherein said kit further comprises a means for detecting said fluorescent probe.

21. The use of the kit of any one of Claims 12 to 20, wherein the use is for the detection of diabetes-induced damage to the nephron.

22. The use of the kit of Claim 21, wherein the diabetes-induced damage to the nephron is correlated with one or more non-molecular indicators of diabetic nephropathy.

23. The use of the kit of any one of Claims 12 to 22, wherein the kit further comprises control DNA indicating expression levels of one of PPARGC1A, SMAD1, UMOD, NRF2, SLC12A1 and CD24 in the absence of diabetic nephropathy.

24. The use of the kit of any one of Claims 12 to 23, wherein the use is for detecting diabetic nephropathy that is due to Type I or Type II diabetes.

## Patentansprüche

1. Verfahren zum Identifizieren eines Individuums, das derzeit von diabetischer Nephropathie aufgrund Diabetes vom Typ I oder Typ II betroffen ist, umfassend:
Isolieren von Vesikeln, die mit RNA assoziiert sind, aus einer Urinprobe, die von einem Individuum erhalten wurde, wobei die Probe Vesikel umfasst, die mit RNA assoziiert sind;
Lysieren der Vesikel, um die Vesikel-assoziierte RNA freizusetzen, wobei die Vesikel-assoziierte RNA eine RNA umfasst, die mit einem Diabetes-induzierten Nephron-Schaden assoziiert ist,
wobei die RNA, die mit dem Diabetes-induzierten Nephron-Schaden assoziiert ist, ausgewählt ist aus der Gruppe bestehend aus PPARGC1A, SMAD1, NRF2, UMOD, SLC12A1 und CD24;
Quantifizieren der RNA, die mit einem Diabetes-induzierten Nephron-Schaden assoziiert ist, durch ein Verfahren, das umfasst:
(i) Inkontaktbringen der RNA aus der Probe mit einer reversen Transkriptase, um eine komplementäre DNA (cDNA) zu erzeugen, und
(ii) Inkontaktbringen der cDNA mit Sense- und Antisense-Primern, die für eines aus PPARGC1A, SMAD1, NRF2, UMOD, SLC12A1 und CD24 spezifisch sind, und einer DNA-Polymerase, um amplifizierte DNA zu erzeugen;
Vergleichen der Menge an quantifizierter RNA von der Probe, die mit einem Diabetes-induzierten Nephron-Schaden assoziiert ist, mit einer Menge an entsprechender RNA aus Individuen, die eine normale Nierenfunktion haben; und
Identifizieren eines Individuums, das derzeit von diabetischer Nephropathie aufgrund Diabetes vom Typ I oder Typ II betroffen ist, wenn es einen Unterschied gibt zwischen der Menge der RNA, die mit einem Diabetes-induzierten Nephron-Schaden bei dem Individuum assoziiert ist, und der Menge an RNA bei Individuen mit normaler Nierenfunktion.

2. Verfahren nach Anspruch 1, wobei der Unterschied der Menge an RNA, die mit einem Diabetes-induzierten Nephron-Schaden assoziiert ist, mit einem oder mehreren nicht-molekularen Indikatoren diabetischer Nephropathie korreliert wird.

3. Verfahren nach Anspruch 1 oder 2, wobei die RNA, die mit einem Diabetes-induzierten Nephron-Schaden assoziiert ist, ausgewählt ist aus der Gruppe bestehend aus PPARGCIA, SMAD1, UMOD und SLC12A1.

4. Verfahren nach einem der Ansprüche 1 bis 3, wobei das Isolieren der Vesikel aus der Probe das Filtern des Urins umfasst, wobei die Filtration die Vesikel auf dem Filter fängt.

5. Verfahren nach Anspruch 4, wobei das Lysieren durchgeführt wird, während die Vesikel auf dem Filter gefangen sind.

6. Verfahren nach einem der Ansprüche 1 bis 5, das des Weiteren das Zentrifugieren der Probe umfasst, um Zelltrümmer zu entfernen, wobei das Zentrifugieren vor dem Isolieren der Vesikel durchgeführt wird.

7. Verfahren nach Anspruch 6, wobei das Konzentrieren der Vesikel des Weiteren das Filtern des Überstandes des zentrifugierten Urins umfasst.

8. Verfahren nach einem der Ansprüche 1 bis 7, wobei der Diabetes vom Typ I oder vom Typ II noch nicht diagnostiziert ist.

9. Verfahren nach einem der Ansprüche 1 bis 8, wobei die RNA, die mit einem Diabetes-induzierten Nephron-Schaden assoziiert ist, poly(A)+ RNA umfasst.

10. Verfahren nach einem der vorangehenden Ansprüche, wobei das Verfahren verwendet wird, um eine Vielzahl an Individuen zu screenen, um deren Wahrscheinlichkeit zu bestimmen, diabetische Nephropathie aufgrund von Diabetes vom Typ I oder vom Typ II zu entwickeln und/oder um diabetische Nephropathie aufgrund von Diabetes vom Typ I oder vom Typ II im Anfangsstadium nachzuweisen.

11. Verfahren zum Identifizieren eines Individuums, das von diabetischer Nephropathie aufgrund Diabetes vom Typ I oder Typ II betroffen ist, umfassend:
Isolieren von Vesikeln, die mit RNA assoziiert sind, aus einer Urinprobe, die von einem Individuum erhalten wurde, wobei die Probe Vesikel umfasst, die mit RNA assoziiert sind;
Lysieren der Vesikel, um die Vesikel-assoziierte RNA freizusetzen, wobei die Vesikel-assoziierte RNA eine RNA umfasst, die mit einem Diabetes-induzierten Nephron-Schaden assoziiert ist,
wobei die RNA, die mit dem Diabetes-induzierten Nephron-Schaden assoziiert ist, ausgewählt ist aus der Gruppe bestehend aus PPARGC1A, SMAD1, NRF2, UMOD, SLC12A1 und CD24;
Quantifizieren der RNA, die mit einem Diabetes-induzierten Nephron-Schaden assoziiert ist, beispielsweise durch Verwendung eines Verfahrens ausgewählt aus der Gruppe bestehend aus RNA-Sequenzierung, RNA-Mikroarray, Nucleinsäuresequenz-basierte Amplifizierung, Northern Blotting, RNase-Schutz-Assay und Verzweigte-DNA-Amplifizierung; und
Vergleichen der Menge der RNA, die mit einem Diabetes-induzierten Nephron-Schaden des Individuums assoziiert ist, mit der Menge an entsprechender RNA aus Individuen, die eine normale Nierenfunktion haben, wobei ein Unterschied der Menge der RNA der Probe, die mit einem Diabetes-induzierten Nephron-Schaden assoziiert ist, zwischen dem Individuum und den Individuen darauf hinweist, dass das Individuum von diabetischer Nephropathie aufgrund von Diabetes vom Typ I oder Typ II betroffen ist.

12. Verwendung eines Kits zum Nachweis von diabetischer Nephropathie im Anfangsstadium, umfassend:
(a) Reverse-Transkriptase-Enzym zum Erzeugen von komplementärer DNA (cDNA) aus RNA, die aus einer Urinprobe von einem Individuum isoliert wurde, das bezüglich seines diabetischen Nephropathie-Status untersucht wird;
(b) mindestens ein Paar an Sense- und Antisense-Primern, die für einen aus PPARGC1A, UMOD, NRF2, SLC12A1 und CD24 spezifisch sind; und
(c) eine DNA-Polymerase zum Erzeugen von amplifizierter DNA aus der cDNA.

13. Verwendung des Kits nach Anspruch 12, wobei der Kit des Weiteren einen Filter zum Einfangen von Vesikeln aus der Urinprobe umfasst.

14. Verwendung des Kits nach Anspruch 13, wobei der Kit des Weiteren einen Lysepuffer zum Freisetzen der RNA von den Vesikeln umfasst.

15. Verwendung des Kits nach Anspruch 14, wobei der Kit des Weiteren einen Elutionspuffer zum Transportieren der RNA von den lysierten Vesikeln zu einem Analysegefäß umfasst.

16. Verwendung des Kits nach einem der Ansprüche 12 bis 15, wobei der Kit des Weiteren eine Mikroplatte umfasst, die konfiguriert ist, um die RNA aufzunehmen.

17. Verwendung des Kits nach Anspruch 16, wobei die Mikroplatte Oligo(dT) in jeder Vertiefung der Platte umfasst.

18. Verwendung des Kits nach einem der Ansprüche 12 bis 17, wobei der Kit des Weiteren einen DNA-Amplifizierungspuffer umfasst, der Tris-HCl, Magnesiumchloride, Natriumchlorid und Adenin-, Thymin-, Guanin- und Cytosin-Nucleotide umfasst.

19. Verwendung des Kits nach einem der Ansprüche 12 bis 18, wobei der Kit des Weiteren mindestens eine Fluoreszenzsonde, die zu einer Region innerhalb der amplifizierten DNA aus einem von PPARGC1A, SMAD1, UMOD, NRF2, SLC12A1 und CD24 umfasst.

20. Verwendung des Kits nach Anspruch 19, wobei der Kit des Weiteren ein Mittel für den Nachweis der Fluoreszenzsonde umfasst.

21. Verwendung des Kits nach einem der Ansprüche 12 bis 20, wobei die Verwendung zum Nachweis eines Diabetes-induzierten Nephron-Schaden ist.

22. Verwendung des Kits nach Anspruch 21, wobei der Diabetes-induzierte Nephron-Schaden mit einem oder mehreren nicht-molekularen Indikatoren diabetischer Nephropathie korreliert wird.

23. Verwendung des Kits nach einem der Ansprüche 12 bis 22, wobei der Kit des Weiteren Kontroll-DNA umfasst, die die Expressionsspiegel von einem aus PPARGC1A, SMAD1, UMOD, NRF2, SLC12A1 und CD24 in der Abwesenheit von diabetischer Nephropathie anzeigt.

24. Verwendung des Kits nach einem der Ansprüche 12 bis 23, wobei die Verwendung zum Nachweis diabetischer Nephropathie aufgrund von Diabetes vom Typ I oder Typ II ist.

## Revendications

1. Procédé pour identifier un sujet actuellement affecté par la néphropathie diabétique due au diabète de type I ou de type II, comprenant :
l'isolement de vésicules qui sont associées à l'ARN d'un échantillon d'urine qui a été obtenu chez un sujet, où ledit échantillon comprend des vésicules qui sont associées à l'ARN ;
la lyse desdites vésicules afin de libérer ledit ARN associé aux vésicules, où ledit ARN associé aux vésicules comprend un ARN associé à une lésion au néphron induite par le diabète,
dans lequel ledit ARN associé à une lésion au néphron induite par le diabète est sélectionné dans le groupe consistant en PPARGC1A, SMAD1, NRF2, UMOD, SLC12A1 et CD24 ;
la quantification dudit ARN associé à une lésion au néphron induite par le diabète par un procédé comprenant :
(i) la mise en contact dudit ARN dudit échantillon avec une transcriptase inverse afin de générer un ADN complémentaire (ADNc), et
(ii) la mise en contact dudit ADNc avec des amorces sens et antisens qui sont spécifiques pour l'un de PPARGC1A, SMAD1, NRF2, UMOD, SLC12A1 et CD24 et une ADN polymérase afin de générer de l'ADN amplifié ;
la comparaison de la quantité d'ARN quantifié associé à une lésion au néphron induite par le diabète dudit échantillon à une quantité d'un ARN correspondant issu d'individus présentant une fonction rénale normale ; et
l'identification d'un sujet actuellement affecté par la néphropathie diabétique due au diabète de type I ou de type II lorsqu'il existe une différence entre la quantité dudit ARN associé à une lésion au néphron induite par le diabète chez ledit sujet et ladite quantité de l'ARN chez les individus présentant une fonction rénale normale.

2. Procédé selon la revendication 1, dans lequel la différence de la quantité d'ARN associé à une lésion au néphron induite par le diabète est corrélée avec un ou plusieurs indicateurs non moléculaires de la néphropathie diabétique.

3. Procédé selon la revendication 1 ou 2, dans lequel ledit ARN associé à une lésion au néphron induite par le diabète est sélectionné dans le groupe consistant en PPARGC1A, SMAD1, UMOD et SLC12A1.

4. Procédé selon l'une quelconque des revendications 1 à 3, dans lequel l'isolement des vésicules dudit échantillon comprend la filtration de l'urine, où ladite filtration piège lesdites vésicules sur le filtre.

5. Procédé selon la revendication 4, dans lequel ladite lyse est réalisée alors que lesdites vésicules sont piégées sur ledit filtre.

6. Procédé selon l'une quelconque des revendications 1 à 5, comprenant en outre la centrifugation dudit échantillon afin d'éliminer les débris cellulaires, où ladite centrifugation est réalisée avant l'isolement des vésicules.

7. Procédé selon la revendication 6, dans lequel la concentration des vésicules comprend en outre la filtration du surnageant de ladite urine centrifugée.

8. Procédé selon l'une quelconque des revendications 1 à 7, dans lequel ledit diabète de type I ou de type II n'a pas encore été diagnostiqué.

9. Procédé selon l'une quelconque des revendications 1 à 8, dans lequel ledit ARN associé à une lésion au néphron induite par le diabète comprend de l'ARN poly(A)+.

10. Procédé selon l'une quelconque des revendications précédentes, où le procédé est utilisé pour examiner une pluralité de sujets afin de déterminer leur probabilité de développer une néphropathie diabétique due au diabète de type I ou de type II et/ou de détecter une néphropathie diabétique de stade précoce due au diabète de type I ou de type II.

11. Procédé pour identifier un sujet affecté par la néphropathie diabétique due au diabète de type I ou de type II, comprenant :
l'isolement de vésicules qui sont associées à l'ARN d'un échantillon d'urine qui a été obtenu chez un sujet, où ledit échantillon comprend des vésicules qui sont associées à l'ARN ;
la lyse desdites vésicules afin de libérer ledit ARN associé aux vésicules, où ledit ARN associé aux vésicules comprend un ARN associé à une lésion au néphron induite par le diabète,
dans lequel ledit ARN associé à une lésion au néphron induite par le diabète est sélectionné dans le groupe consistant en PPARGC1A, SMAD1, NRF2, UMOD, SLC12A1 et CD24 ;
la quantification dudit ARN associé à une lésion au néphron induite par le diabète tel qu'en utilisant un procédé sélectionné dans le groupe consistant en un séquençage d'ARN, un microréseau d'ARN, une amplification basée sur une séquence d'acide nucléique, un transfert de Northern, un essai de protection à la RNase et une amplification d'ADN branché ; et
la comparaison de la quantité dudit ARN associé à une lésion au néphron induite par le diabète dudit sujet à la quantité d'un ARN correspondant issu d'individus présentant une fonction rénale normale, où une différence de la quantité dudit ARN associé à une lésion au néphron induite par le diabète entre ledit sujet et lesdits individus indique que le sujet est affecté par la néphropathie diabétique due au diabète de type I ou de type II.

12. Utilisation d'un kit pour la détection d'une néphropathie diabétique de stade précoce, comprenant :
(a) une enzyme transcriptase inverse pour générer un ADN complémentaire (ADNc) à partir d'ARN isolé d'un échantillon d'urine d'un sujet qui est évalué pour son statut de néphropathie diabétique ;
(b) au moins une paire d'amorces sens et antisens spécifiques pour l'un de PPARGC1A, UMOD, NRF2, SLC12A1 et CD24 ; et
(c) une ADN polymérase pour générer de l'ADN amplifié à partir de l'ADNc.

13. Utilisation du kit selon la revendication 12, dans laquelle ledit kit comprend en outre un filtre pour capturer des vésicules à partir dudit échantillon d'urine.

14. Utilisation du kit selon la revendication 13, dans laquelle ledit kit comprend en outre un tampon de lyse pour libérer l'ARN desdites vésicules.

15. Utilisation du kit selon la revendication 14, dans laquelle ledit kit comprend en outre un tampon d'élution pour transporter l'ARN desdites vésicules lysées vers un récipient d'analyse.

16. Utilisation du kit selon l'une quelconque des revendications 12 à 15, dans laquelle ledit kit comprend en outre une microplaque configurée pour recevoir ledit ARN.

17. Utilisation du kit selon la revendication 16, dans laquelle ladite microplaque comprend un oligo(dT) dans chaque puits de la plaque.

18. Utilisation du kit selon l'une quelconque des revendications 12 à 17, dans laquelle ledit kit comprend en outre un tampon d'amplification d'ADN comprenant du Tris-HCl, du chlorure de magnésium, du chlorure de potassium, et les nucléotides adénine, thymine, guanine, et cytosine.

19. Utilisation du kit selon l'une quelconque des revendications 12 à 18, dans laquelle ledit kit comprend en outre au moins une sonde fluorescente complémentaire à une région au sein de l'ADN amplifié de l'un de PPARGC1A, SMAD1, UMOD, NRF2, SLC12A1 et CD24.

20. Utilisation du kit selon la revendication 19, dans laquelle ledit kit comprend en outre un moyen pour détecter ladite sonde fluorescente.

21. Utilisation du kit selon l'une quelconque des revendications 12 à 20, dans laquelle l'utilisation est pour la détection d'une lésion au néphron induite par le diabète.

22. Utilisation du kit selon la revendication 21, dans laquelle la lésion au néphron induite par le diabète est corrélée avec un ou plusieurs indicateurs non moléculaires de la néphropathie diabétique.

23. Utilisation du kit selon l'une quelconque des revendications 12 à 22, dans laquelle le kit comprend en outre un ADN de contrôle indiquant les taux d'expression de l'un de PPARGC1A, SMAD1, UMOD, NRF2, SLC12A1 et CD24 en l'absence de néphropathie diabétique.

24. Utilisation du kit selon l'une quelconque des revendications 12 à 23, dans laquelle l'utilisation est pour détecter une néphropathie diabétique qui est due au diabète de type I ou de type II.


### SEQUENCE LISTING

<110> Hitachi Chemical Company, Ltd. Hitachi Chemical Research Center, Inc. The Regents of the University of California
<120> URINE EXOSOME mRNAs AND METHODS OF USING SAME TO DETECT DIABETIC NEPHROPATHY
<130> HITACHI.114WO
<150> 61/710,627
   <151> 2012-10-05
<160> 44
<170> PatentIn version 3.5
<210> 1
   <211> 18
   <212> DNA
   <213> Homo sapiens
<220>
   <221> misc_feature
   <223> Beta Actin Forward Primer
<400> 1
   cctggcaccc agcacaat 18
<210> 2
   <211> 20
   <212> DNA
   <213> Homo sapiens
<220>
   <221> misc_feature
   <223> Beta Actin Reverse Primer
<400> 2
   gccgatccac acggagtact 20
<210> 3
   <211> 23
   <212> DNA
   <213> Homo sapiens
<220>
   <221> misc_feature
   <223> Beta-2 Microgloublin Forward Primer
<400> 3
   tgactttgtc acagcccaag ata 23
<210> 4
   <211> 20
   <212> DNA
   <213> Homo sapiens
<220>
   <221> misc_feature
   <223> Beta-2 Microglobulin Reverse Primer
<400> 4
   aatgcggcat cttcaaacct 20
<210> 5
   <211> 22
   <212> DNA
   <213> Homo sapiens
<220>
   <221> misc_feature
   <223> Glomerulus Specific Podocin Forward Primer
<400> 5
   aggatggcag ctgagattct gt 22
<210> 6
   <211> 24
   <212> DNA
   <213> Homo sapiens
<220>
   <221> misc_feature
   <223> Glomerulus Specific Podocin Reverse Primer
<400> 6
   agagactgaa gggtgtggag gtat 24
<210> 7
   <211> 20
   <212> DNA
   <213> Homo sapiens
<220>
   <221> misc_feature
   <223> Uromodulin Forward Primer
<400> 7
   cctgaacttg ggtcccatca 20
<210> 8
   <211> 19
   <212> DNA
   <213> Homo sapiens
<220>
   <221> misc_feature
   <223> Uromodulin Reverse Primer
<400> 8
   gccccaagct gctaaaagc 19
<210> 9
   <211> 20
   <212> DNA
   <213> Homo sapiens
<220>
   <221> misc_feature
   <223> Albumin Forward Primer
<400> 9
   tgcaaggctg acgataagga 20
<210> 10
   <211> 26
   <212> DNA
   <213> Homo sapiens
<220>
   <221> misc_feature
   <223> Albumin Reverse Primer
<400> 10
   gtaggctgag atgcttttaa atgtga 26
<210> 11
   <211> 25
   <212> DNA
   <213> Homo sapiens
<220>
   <221> misc_feature
   <223> Na+/K+/Cl- transporter Forward Primer
<400> 11
   actccagagc tgctaatctc attgt 25
<210> 12
   <211> 26
   <212> DNA
   <213> Homo sapiens
<220>
   <221> misc_feature
   <223> Na+/K+/Cl- transporter Reverse Primer
<400> 12
   aactagtaag acaggtggga ggttct 26
<210> 13
   <211> 25
   <212> DNA
   <213> Homo sapiens
<220>
   <221> misc_feature
   <223> Distal tubule specific aquaporin 9 Forward Primer
<400> 13
   aaacaacttc tggtggattc ctgta 25
<210> 14
   <211> 21
   <212> DNA
   <213> Homo sapiens
<220>
   <221> misc_feature
   <223> Distal tubule specific aquaporin 9 Reverse Primer
<400> 14
   gctctggatg gtggatttca a 21
<210> 15
   <211> 25
   <212> DNA
   <213> Homo sapiens
<220>
   <221> misc_feature
   <223> Peroxisome proliferator-activated receptor gamma, coactivator 1 alpha Forward Primer
<400> 15
   gctcttgaaa atggatacac tttgc 25
<210> 16
   <211> 27
   <212> DNA
   <213> Homo sapiens
<220>
   <221> misc_feature
   <223> Peroxisome proliferator-activated receptor gamma, coactivator 1 alpha Reverse Primer
<400> 16
   tctgagtttg aatctaggtc tgcatag 27
<210> 17
   <211> 21
   <212> DNA
   <213> Homo sapiens
<220>
   <221> misc_feature
   <223> Peroxisome proliferator-activated receptor gamma, coactivator 1 Beta Forward Primer
<400> 17
   cccttctcct gttcctttgg a 21
<210> 18
   <211> 19
   <212> DNA
   <213> Homo sapiens
<220>
   <221> misc_feature
   <223> Peroxisome proliferator-activated receptor gamma, coactivator 1 Beta Reverse Primer
<400> 18
   cctttgcagg acgccttct 19
<210> 19
   <211> 22
   <212> DNA
   <213> Homo sapiens
<220>
   <221> misc_feature
   <223> Nuclear respiratory factor 1 Forward Primer
<400> 19
   ccagatccct gtgagcatgt ac 22
<210> 20
   <211> 22
   <212> DNA
   <213> Homo sapiens
<220>
   <221> misc_feature
   <223> Nuclear respiratory factor 1 Reverse Primer
<400> 20
   tgactgcgct gtctgatatc ct 22
<210> 21
   <211> 23
   <212> DNA
   <213> Homo sapiens
<220>
   <221> misc_feature
   <223> Nuclear respiratory factor 2 Forward Primer
<400> 21
   catgctacgt gatgaagatg gaa 23
<210> 22
   <211> 23
   <212> DNA
   <213> Homo sapiens
<220>
   <221> misc_feature
   <223> Nuclear respiratory factor 2 Reverse Primer
<400> 22
   aacaaggaaa acattgccat ctc 23
<210> 23
   <211> 22
   <212> DNA
   <213> Homo sapiens
<220>
   <221> misc_feature
   <223> Estrogen-related receptor alpha Forward Primer
<400> 23
   aaagtgctgg cccatttcta tg 22
<210> 24
   <211> 22
   <212> DNA
   <213> Homo sapiens
<220>
   <221> misc_feature
   <223> Estrogen-related receptor alpha Reverse Primer
<400> 24
   tctccaagaa cagcttgtgc at 22
<210> 25
   <211> 22
   <212> DNA
   <213> Homo sapiens
<220>
   <221> misc_feature
   <223> Annexin 5 Forward Primer
<400> 25
   tggtttccag gagtgagatt ga 22
<210> 26
   <211> 23
   <212> DNA
   <213> Homo sapiens
<220>
   <221> misc_feature
   <223> Annexin 5 Reverse Primer
<400> 26
   tggaataaag agaggtggca aaa 23
<210> 27
   <211> 21
   <212> DNA
   <213> Homo sapiens
<220>
   <221> misc_feature
   <223> AMP-activated, alpha 1 catalytic subunit Forward Primer
<400> 27
   tcagatgctg aggctcaagg a 21
<210> 28
   <211> 22
   <212> DNA
   <213> Homo sapiens
<220>
   <221> misc_feature
   <223> AMP-activated, alpha 1 catalytic subunit Forward Primer
<400> 28
   tgtgtgactt ccaggtcttg ga 22
<210> 29
   <211> 23
   <212> DNA
   <213> Homo sapiens
<220>
   <221> misc_feature
   <223> AMP-activated, alpha 2 catalytic subunit Forward Primer
<400> 29
   ctgcagagag ccattcactt tct 23
<210> 30
   <211> 24
   <212> DNA
   <213> Homo sapiens
<220>
   <221> misc_feature
   <223> AMP-activated, alpha 2 catalytic subunit Forward Primer
<400> 30
   ggtgaaactg aagacaatgt gctt 24
<210> 31
   <211> 20
   <212> DNA
   <213> Homo sapiens
<220>
   <221> misc_feature
   <223> Uncoupling protein 1 Forward Primer
<400> 31
   ggaccaacgg ctttcttcaa 20
<210> 32
   <211> 22
   <212> DNA
   <213> Homo sapiens
<220>
   <221> misc_feature
   <223> Uncoupling protein 1 Reverse Primer
<400> 32
   cataatgacg ttccaggatc ca 22
<210> 33
   <211> 19
   <212> DNA
   <213> Homo sapiens
<220>
   <221> misc_feature
   <223> Uncoupling protein 2 Forward Primer
<400> 33
   gcttgggttc ctggaacgt 19
<210> 34
   <211> 19
   <212> DNA
   <213> Homo sapiens
<220>
   <221> misc_feature
   <223> Uncoupling protein 2 Forward Primer
<400> 34
   agccatgagg gctcgtttc 19
<210> 35
   <211> 21
   <212> DNA
   <213> Homo sapiens
<220>
   <221> misc_feature
   <223> Low density lipoprotein receptor-related protein 2 Forward Primer
<400> 35
   gcacagatgg agaacgagca a 21
<210> 36
   <211> 19
   <212> DNA
   <213> Homo sapiens
<220>
   <221> misc_feature
   <223> Low density lipoprotein receptor-related protein 2 Reverse Primer
<400> 36
   agcagggagc gaaggtgat 19
<210> 37
   <211> 22
   <212> DNA
   <213> Homo sapiens
<220>
   <221> misc_feature
   <223> CD24 Forward Primer
<400> 37
   gacactcccc gaagtctttt gt 22
<210> 38
   <211> 27
   <212> DNA
   <213> Homo sapiens
<220>
   <221> misc_feature
   <223> CD24 Reverse Primer
<400> 38
   tcatcaagac tactgtggcc atattag 27
<210> 39
   <211> 22
   <212> DNA
   <213> Homo sapiens
<220>
   <221> misc_feature
   <223> Secreted Phosphoprotein 1 Forward Primer
<400> 39
   agccaatgat gagagcaatg ag 22
<210> 40
   <211> 21
   <212> DNA
   <213> Homo sapiens
<220>
   <221> misc_feature
   <223> Secreted phosphoprotein 1 Reverse Primer
<400> 40
   tggaattcac ggctgacttt g 21
<210> 41
   <211> 20
   <212> DNA
   <213> Homo sapiens
<220>
   <221> misc_feature
   <223> Alpha 2 HS-glycoprotein Forward Primer
<400> 41
   catgggtgtg gtctcattgg 20
<210> 42
   <211> 22
   <212> DNA
   <213> Homo sapiens
<220>
   <221> misc_feature
   <223> Alpha 2 HS-glycoprotein Reverse Primer
<400> 42
   caacactagg ctgcaccact gt 22
<210> 43
   <211> 25
   <212> DNA
   <213> Homo sapiens
<220>
   <221> misc_feature
   <223> SMAD family member 1
<220>
   <221> misc_feature
   <223> SMAD family member 1 Forward Primer
<400> 43
   ctgctattct gaaattgcct acatg 25
<210> 44
   <211> 30
   <212> DNA
   <213> Homo sapiens
<220>
   <221> misc_feature
   <223> SMAD family member 1 Reverse Primer
<400> 44
   actgtaaact ccgtaaaaac tgcttattaa 30